(19) **Europäisches Patentamt**

**European Patent Office**

**Office européen des brevets**

(11) Publication number : **0 446 710 B1**

(12) **EUROPEAN PATENT SPECIFICATION**

(45) Date of publication of patent specification :
29.12.93 Bulletin 93/52

(51) Int. Cl.$^5$ : **B01J 23/89,** B01J 37/00, C10G 2/00, C07C 1/12, C22C 1/00

(21) Application number : **91102920.5**

(22) Date of filing : **27.02.91**

(54) **The use of an amorphous alloy catalyst for conversion of carbon dioxide.**

(30) Priority : **28.02.90 JP 45660/90**

(43) Date of publication of application :
**18.09.91 Bulletin 91/38**

(45) Publication of the grant of the patent :
**29.12.93 Bulletin 93/52**

(84) Designated Contracting States :
**CH DE ES FR GB IT LI SE**

(56) References cited :
**EP-A- 0 173 088**
**EP-A- 0 213 708**
**EP-A- 0 292 184**
**US-A- 2 692 274**
**US-A- 4 717 696**

(73) Proprietor : **YOSHIDA KOGYO K.K.**
**No. 1 Kanda Izumi-cho Chiyoda-ku**
**Tokyo (JP)**
Proprietor : **Hashimoto, Koji**
**2-25-5, Shogen Izumi-ku**
**Sendai-shi Miyagi (JP)**

(72) Inventor : **Hashimoto, Koji**
**2-25-5, Shogen, Izumi-ku, Sendai-shi,**
**Miyagi (JP)**
Inventor : **Wakuda, Kenichiro**
**1519, Hachigata, Kashima-machi,**
**Kashima-gun**
**Ibaraki (JP)**
Inventor : **Habazaki, Hiroki**
**101 Midoriso, 2-13-26, Kunimi, Aoba-ku,**
**Sendai-shi**
**Miyagi (JP)**

(74) Representative : **Patentanwälte Leinweber &**
**Zimmermann**
**Rosental 7/II Aufg.**
**D-80331 München (DE)**

## Description

BACKGROUND OF THE INVENTION

1. Field of the Invention

The present invention relates to materials which are particularly suitable as highly active catalysts for conversion of carbon dioxide to hydrocarbons and are characterized by easy production of catalysts and easy recovery of its catalytically useful components.

2. Description of the Prior Art

Catalysts for the conversion of carbon monoxide to hydrocarbon by hydrogenation have been well investigated, and well known catalysts are ruthenium supported by alumina.

In general, ordinary alloys are crystalline in the solid state. However, rapid quenching of some alloys with specific compositions from the liquid state gives rise to the solidification to an amorphous structure. These alloys are called amorphous alloys. The amorphous alloys have significantly high mechanical strength in comparison with conventional industrial alloys. Some amorphous alloys with specific compositions have a variety of superior characteristics including extremely high corrosion resistance that cannot be obtained in ordinary crystalline alloys. Amorphous Ni-Zr and Pd-Zr alloys are active at low temperatures such as 250-300 °C as catalysts for conversion of carbon monoxide to hydrocarbon by hydrogenation and are several times more active than the ruthenium catalyst supported by alumina.

One of the present inventors and co-inventors applied Japanese Patent Application No. 123111/85 of amorphous alloy electrode materials containing Ni, Ta and platinum group elements as essential components which are suitable for as an anode for oxygen production by the electrolysis of acidic aqueous solutions because of their high activity for oxygen evolution.

Japanese Patent Application No. 123111/85 discloses:

(1) Amorphous alloy electrode materials which comprise 25 to 65 at% Ta, 0.3 to 45 at% of at least one element selected from the group of Ru, Rh, Pd, Ir and Pt, and more than 30 at% Ni.

(2) Amorphous alloy electrode materials which comprise 25 to 65 at% in the total of 20 at% or more Ta and at least one element selected from the group of Ti, Zr and Nb, 0.3 to 45 at% at least one element selected from the group of Ru, Rh, Pd, Ir and Pt, and more than 30 at% Ni.

Three of the present inventors further invented surface-activated amorphous alloy electrode materials suitable for electrolysis of aqueous solutions, consisting of Ni, very small amounts of platinum group metals and at least one element selected from the group of Ti, Zr, Nb and Ta, and their activation methods, and applied Japanese Patent Application Nos. 169764/85, 169765/85 and 169767/85. They further applied Japanese Patent Application No. 169766/85 for surface-activated supersaturated solid solution alloy electrode materials for electrolysis of aqueous solutions and their activation methods.

Japanese Patent Application No. 169764/85 discloses:

(1) Surface-activated amorphous alloy electrode materials consisting of 25 to 65 at% of Nb, 0.01 to 10 at% of at least one element selected from the group of Ru, Rh, Pd, Ir and Pt, and the substantial balance of Ni, to which surface activation treatment was applied for electrolysis of aqueous solutions.

(2) Surface-activated amorphous alloy electrode materials consisting of 25 to 65 at% in the total of 10 at% or more Nb and at least one element selected from the group of Ti, Zr and less than 20 at% of Ta, and 0.01 to 10 at% of at least one element selected from the group of Ru, Rh, Pd, Ir and Pt, and the substantial balance of Ni, to which surface activation treatment was applied for electrolysis of aqueous solutions.

(3) Surface-activated amorphous alloy electrode materials consisting of 25 to 65 at% of Nb, 0.01 to 10 at% of at least one element selected from the group consisting of Ru, Rh, Pd, Ir and Pt, less than 7 at% of P and the substantial balance of Ni, to which surface activation treatment was applied for electrolysis of aqueous solutions.

(4) Surface-activated amorphous alloy electrode materials consisting of 25 to 65 at% in the total of 10 at% or more Nb and at least one element selected from the group consisting of Ti, Zr and less than 20 at% of Ta, and 0.01 to 10 at% of at least one element selected from the group of Ru, Rh, Pd, Ir and Pt, less than 7 at% of P and the substantial balance of Ni, to which surface activation treatment was applied for electrolysis of aqueous solutions.

(5) A method for the activation treatment of amorphous alloys for electrodes characterized by the immersion of the above-mentioned amorphous alloy electrode materials into corrosive solutions for surface enrichment of platinum group elements as a result of preferential dissolution of Ni, Nb, Ta, Ti and Zr.

Japanese Patent-Application No. 169765/85 discloses:

(1) Surface-activated amorphous alloy electrode materials consisting of 25 to 65 at% of Ta, 0.01 to 10 at% of at least one element selected from the group consisting of Ru, Rh, Pd, Ir and Pt, less than 7 at% of P and the substantial balance of 20 at% or more Ni, to which surface activation treatment was applied for electrolysis of aqueous solutions.

(2) Surface-activated amorphous alloy electrode materials consisting of 25 to 65 at% in the total of 20 at% or more Ta and at least one element selected from the group of Ti, Zr and Nb, 0.01 to 10 at% of at least one element selected from the group of Ru, Rh, Pd, Ir and Pt, less than 7 at% of P and the substantial balance of 20 at% or more Ni, to which surface activation treatment was applied for electrolysis of aqueous solutions.

(3) A method for the activation treatment of amorphous alloys for electrodes characterized by the immersion of the above-mentioned amorphous alloy electrode materials into corrosive solutions for surface enrichment of platinum group elements as a result of preferential dissolution of Ni, Nb, Ta, Ti and Zr.

Japanese Patent Application No. 169767/85 discloses:

(1) Surface-activated amorphous alloy electrode materials consisting of 25 to 65 at% in the total of 5 at% to less than 20 at% of Ta and at least one element selected from the group of Ti and Zr, and 0.01 to 10 at% of at least one element selected from the group of Ru, Rh, Pd, Ir and Pt, and the substantial balance of Ni, to which surface activation treatment was applied for the electrolysis of aqueous solutions.

(2) Surface-activated amorphous alloy electrode materials consisting of 25 to 65 at% in the total of 5 at% to less than 20 at% of Ta and at least one element selected from the group of Ti and Zr, and 0.01 to 10 at% of at least one element selected from the group of Ru, Rh, Pd, Ir and Pt, less than 7 at% of P and the substantial balance of 20 at% or more Ni, to which surface activation treatment was applied for the electrolysis of aqueous solutions.

(3) A method for the activation treatment of amorphous alloys for electrodes characterized by the immersion of the above-mentioned amorphous alloy electrode materials into corrosive solutions for surface enrichment of platinum group elements as a result of the preferential dissolution of Ni, Ta, Ti and Zr.

Japanese Patent Application No. 169766/85 discloses:

(1) Surface-activated supersaturated solid solution alloy electrode materials consisting of 20 to less than 25 at% of at least one element selected from the group of Nb and Ta, 0.01 to 10 at% of at least one element selected from the group of Ru, Rh, Pd, Ir and Pt, and the substantial balance of Ni, to which surface activation treatment was applied for electrolysis of aqueous solutions.

(2) Surface-activated supersaturated solid solution alloy electrode materials consisting of 20 to less than 25 at% of at least one element selected from the group of Nb and Ta, 0.01 to 10 at% of at least one element selected from the group of Ru, Rh, Pd, Ir and Pt, less than 7 at% of P, and the substantial balance of Ni, to which surface activation treatment was applied for electrolysis of aqueous solutions.

(3) surface-activated supersaturated solid solution alloy electrode materials consisting of 20 to less than 25 at% in the total of at least one element selected from the group of Ti and Zr and 5 at% or more of at least one element selected from the group of Nb and Ta, 0.01 to 10 at% of at least one element selected from the group of Ru, Rh, Pd, Ir and Pt, and the substantial balance of Ni, to which surface activation treatment was applied for electrolysis of aqueous solutions.

(4) Surface-activated supersaturated solid solution alloy electrode materials consisting of 20 to less than 25 at% in the total of at least one element selected from the group of Ti and Zr and 5 at% or more of at least one element selected from the group of Nb and Ta, 0.01 to 10 at% of at least one element selected from the group of Ru, Rh, Pd, Ir and Pt, less than 7 at% of P, and the substantial balance of Ni, to which surface activation treatment was applied for electrolysis of aqueous solutions.

(5) A method for the activation treatment of supersaturated solid solution alloys for electrodes characterized by the immersion of the above-mentioned supersaturated solid solution alloy electrode materials into corrosive solutions for surface enrichment of platinum group elements as a result of the preferential dissolution of Ni, Nb, Ta, Ti and Zr.

Furthermore, the present inventors found surface-activated amorphous alloys for methanol fuel cell and made application of Japanese Patent Application No.154570/86.

Japanese Patent Application no. 154570/86 discloses:

(1) Surface-activated amorphous alloys for methanol fuel cells, consisting of 20 to 80 at% of at least one element selected from the group of Ti and Zr, 0.5 to 20 at% of Pt, and the substantial balance of 10 at% or more of at least one element selected from the group of Ni and Co.

(2) Surface-activated amorphous alloys for methanol fuel cells, consisting of 20 to 80 at% of at least one element selected from the group of Ti and Zr, 0.5 to 20 at% of Pt, at most 10 at% (at most the same at% as Pt if Pt is at most 10 at%) of at least one element selected from the group of Ru, Rh, Pd, Ir, Tl, Si, Ge, Sn, Pb and Bi, and the substantial balance of 10 at% or more of at least one element selected from the group of Ni and Co.

(3) Surface-activated amorphous alloys for methanol fuel cells, consisting of 20 to 70 at% of at least one element selected from the group of Nb and Ta, 0.5 to at% of Pt, and the substantial balance of at least one element selected from the group of Ni and Co.

(4) Surface-activated amorphous alloys for methanol fuel cells, consisting of 20 to 70 at% of at of at least one element selected from the group of Nb and Ta, 0.5 to 20 at% of Pt, at most 10 at% (at most the same at% as Pt if Pt is at most 10 at%) of at least one element selected from the group of Ru, Rh, Pd, Ir, Tl, Si, Ge, Sn, Pb and Bi, and the substantial balance of 10 at% or more of at least one element selected from the group of Ni and Co.

(5) Supface-activated amorphous alloys for methanol fuel cells consisting of 20 to 80 at% in the total of at least one element selected from the group of Ti and Zr and at most 70 at% of at least one element selected from the group of Nb and Ta, 0.5 to 20 at% of Pt, and the substantial balance of 10 at% or more of at least one element selected from the group of Ni and Co.

(6) Surface-activated amorphous alloy for methanol fuel cell, consisting of 20 to 80 at% in the total of at least one element selected from the group of Ti and Zr and at most 70 at% of at least one element selected from the group of Nb and Ta, 0.5 to 20 at% of Pt, at most 10 at% (at most the same at% as Pt if Pt is at most 10 at%) of at least one element selected from the group of Ru, Rh, Pd, Ir, Tl, Si, Ge, Sn, Pb and Bi, and the substantial balance of 10 at% or more of at least one element selected from the group of Ni and Co.

In order to overcome the high operation temperatures of catalysts consisting of platinum group elements supported on ceramics used in the purification of exhaust gases from plants and vehicles, in addition to the difficulty of recovery of platinum group elements, three of the preset inventors investigated catalysts capable of operating at low temperatures, such as the beginning of combustion, in addition to easy recovery, and invented catalysts for the purification of exhaust gases by the reaction of carbon monoxide with nitrogen oxides in exhaust gases as Japanese Patent Application No. 262986/89.

Japanese Patent Application No. 262986/89 discloses:

(1) Surface-activated catalysts for the purification of exhaust gases, consisting of 20 to 70 at% of at least one element selected from the group of Nb and Ta, 0.5 to 20 at% of at least one element selected from the group of Ru, Rh, Pd, Ir and Pt, and the substantial balance of at least one element selected from the group of Ni and Co, to which surface activation treatment was applied.

(2) surface-activated catalysts for the purification of exhaust gases, consisting of 20 to 80 at% of at least one element selected from the group of Ti and Zr, 0.5 to 20 at% of at least one element selected from the group of Ru, Rh, Pd, Ir and Pt, and the substantial balance of at least 10 at% of at least one element selected from the group of Ni and Co, to which surface activation treatment was applied.

(3) Surface-activated catalysts for the purification of exhaust gases, consisting of 20 to 80 at% in the total of at least one element selected from the group of Ti and Zr and at most 70 at% of at least one element selected from the group of Nb and Ta, 0.5 to 20 at% of at least one element selected from the group of Ru, Rh, Pd, Ir and Pt, and the substantial balance of at least 10 at% of at least one element selected from the group of Ni and Co, to which surface activation treatment was applied.

Greenhouse effect by a large amount of exhaust carbon dioxide becomes a serious problem. It is however, difficult to decrease the amount of exhaust carbon dioxide without lowering the level of civilian life. It is, therefore, necessary to develop a system by which carbon dioxide can be converted to hydrocarbon for re-use without consumption of a large amount of energy.

In view of the above-foregoing, there has been a strong demand for highly active and easily recoverable catalysts for conversion of carbon dioxide to hydrocarbon at low temperatures with a low consumption of energy.

SUMMARY OF THE INVENTION

It is an objective of the present invention to provide highly active and easily producible and recoverable catalysts by which hydrocarbons are produced by the reaction of carbon dioxide with hydrogen at relatively low temperatures close to ambient temperature.

The objective of the invention has been achieved by finding that some amorphous alloys have a high catalytic activity for the conversion of carbon dioxide. The present invention is composed of a sole claim in which catalytically active and easily producible and recoverable alloys consist of at least one element selected from the group of Co and Ni, valve metals to form the amorphous structure by alloying with at least one element selected from the group of Ni and Co, and a very small amount of platinum group elements effective as the catalyst for the reaction of carbon dioxide with hydrogen, the alloys being activated by hydrofluoric acid treatment.

What is claimed according to the invention is:

Use of a catalyst for conversion of carbon dioxide, said catalyst being produced by
a) providing an amorphous alloy consisting of
- 20 to 70 at% of at least one element selected from the group of Nb and Ta, or 20 to 80 at% of at least one element selected from the group of Ti and Zr, or 20 to 80 at% in the total of at least one element selected from the group of Ti and Zr and at most 70 at% of one element selected from the group of Nb and Ta,
- 0.5 to 20 at% of at least one element selected from the platinum group, and
- the substantial balance of at least one element selected from the group of Ni and Co; and
b) immersing said alloy in hydrofluoric acids for activation.

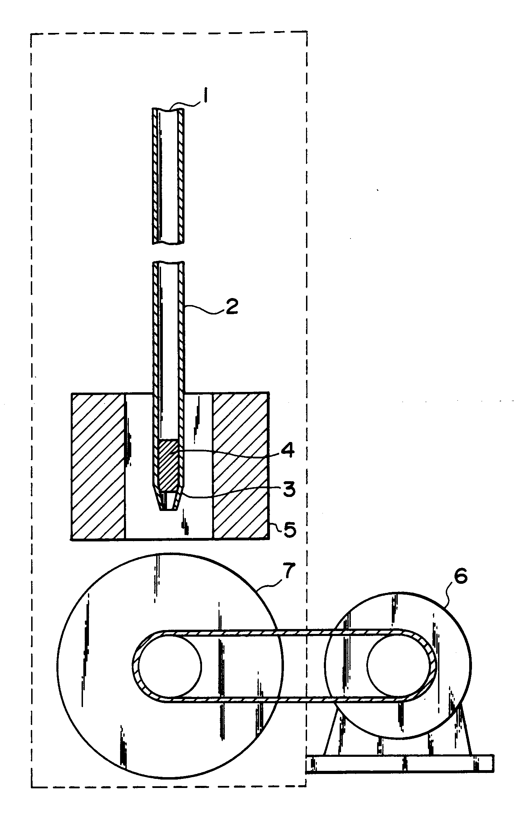

## BRIEF DESCRIPTION OF THE DRAWING

The single figure shows an apparatus for preparing amorphous alloys of the present invention.

## DETAILED DESCRIPTION OF THE PREFERRED EMBODIMENTS

In order to obtain easily producible and recoverable catalysts with a selectively high catalytic activity for a specific reaction, it is more convenient to use alloys containing necessary amounts of effective elements rather than usage of platinum group elements supported by alumina, titania, silica, etc. However, when conventionally processed crystalline alloys are used, the additions of large amounts of various elements often lead to formation of multiple phases of different chemical properties and
to poor mechanical strength, and it is difficult to obtain materials with a large specific surface area necessary for effective catalysts.

On the other hand, the amorphous alloys of the present invention are prepared by the principle to prevent localization of alloy constituents due to rapid quenching of the melt of the above-mentioned compositions and hence, they are highly homogeneous and mechanically strong and tough. When these alloys are treated by immersion in hydrofluoric acids, alloy constituents not necessary for catalytic activity are dissolved into the hydrofluoric acids with the subsequent formation of a highly active catalysts with a remarkably large surface area enriched in catalytically active platinum group elements. In this treatment, hydrogen evolution occurs violently on the platinum group elements in the alloys, since the platinum group elements act as the cathode in the amorphous alloys composed of a chemically homogeneous single phase solid solution. Since the hydrogen evolution provides dissolution of alloy constituents not necessary for catalytic activity, violent hydrogen evolution on the homogeneous alloys results in the rapid and uniform dissolution of alloy constituents not necessary for the catalytic activity. This leads to the formation of highly active catalysts with a large surface area enriched in catalytically active platinum group elements.

Consequently, catalysts for production of hydrocarbons by the reaction of carbon dioxide with hydrogen can be obtained by the alloys of the present invention, to which activation treatment by immersion in hydrofluoric acids is applied.

The components and compositions of the alloys of the present invention are specified as above for the following reactions:

In the alloys of the present invention, Ni and Co are basic components which form the amorphous structure when they coexist with one or more elements selected from the group of Nb, Ta, Ti and Zr. Therefore, in order to form the amorphous structure, the alloys may consists of at least one element selected from the group of Ni and Co and at least one element selected from Nb and Ta should contain 20 to 70 at% of at least one element selected from the group of Nb and Ta. The armorphous structure can also be obtained when the alloys consist of at least one element selected from the group of Ni and Co and at least one element selected from Ti and Zr with 20 to 80 at% of at least one element selected from the group of Ti and Zr.

Furthermore, in the alloys set forth in Claim 3 consisting of at least one element selected from the group of Ni and Co, at least one element selected from Ti and Zr, and at least one element selected from Nb and Ta, the amorphous structure can be obtained if the alloys contain 20 to 80 at% in the total of at least one element selected from the group of Ti and Zr and at most 70 at% of at least one element selected from the group of Nb and Ta.

The platinum group elements Ru, Rh, Pd, Ir and Pt are all effective for high catalytic activity and, hence, the catalytic activity requires that one or more of these platinum group elements should be 0.5 at% or more. However, the additions of large amounts of platinum group elements make the alloys quite expensive and are

5

rather detrimental for both increasing surface area and surface enrichment of the platinum group elements since the beneficial effect of platinum group elements for preferential dissolution of elements not necessary for catalytic activity rather decreases by the excessive addition of platinum group elements. Therefore, the content of platinum group elements shoulds not exceed 20 at%, and the most suitable contents are from 1 to 10 at%.

The preparation of the amorphous alloys of the present invention can be carried out by any kind of method for the preparation of amorphous allys, such as rapid quenching from the liquid state.

One embodiment of an apparatus for preparing the amorphous alloys of the present invention is shown in the accompanying drawing. This is called the rotating wheel method. The apparatus is placed in a vacuum chamber indicated by a dotted rectangle. In the figure, a quartz tube 2 has a nozzle 3 at its lower end in the vertical direction, and raw materials 4 and an inert gas for preventing oxidation of the raw materials are fed from an inlet 1. A heating furnace 5 is placed around the quartz tube 2 so as to heat the raw materials 4. A high speed wheel 7 is placed below the nozzle 3 and is rotated by a motor 6.

For the preparation of the amorphous alloys, the raw materials 4 of the prescribed compositions are placed in the quarts tube 2 and the vacuum chamber is evacuated up to about $10^{-5}$ torr. After the evacuated vacuum chamber is filled with argon gas of about 1 atm, the raw materials 4 are melted by the heating furnace 5. The molten alloy impinges under the pressure of the inert gas onto the outer surface of the wheel 7, which is rotated at a speed of 1,000 to 10,000 rpm, whereby an amorphous alloy is formed as a long thin plate, which may for example have a thickness of 0.01 mm, a width of 10 mm and a length of several meters.

The amorphous alloys of the present invention will be further illustrated by certain examples which are provided only for purpose of illustration and are not intended to limit the present invention.

Example 1

A raw alloy was prepared by argon arc melting of a mixture of commercial metals so as to form Ni-40 at% Zr-1.5 at% Rh-1.5 at% Ru. After remelting of the raw alloy under an argon atmosphere, amorphous alloy ribbons were prepared by the rotating wheel method using the apparatus shown in the figure. The amorphous alloys thus prepared were 0.01-0.05 mm thick, 1-3 mm wide and 3-20 m long ribbons. The formation of an morphous structure was confirmed by X-ray diffraction.

The highly active metallic catalyst was obtained by surface activation treatment of this alloy by immersion in 1.2 % HF at ambient temperature. The reactor tube was prepared by placing 0.5 g of the metallic catalysts of 5 cm length in a glass tube of 8 mm inner diameter and was placed in an electric furnace. The reactant gas consisting of 4.96 % of $CO_2$ and 20 % of $H_2$ in $N_2$ was passed through the reactor tube with a flow rate of 15 ml/min. The amounts of $CO_2$, $CH_4$ and $C_2H_4$ in the gas passing through the reactor tube were analyzed by gas chromatography.

Table 1 shows the reaction temperature and conversion.

## Table 1

| Reaction Temperature °C | Mass of $CO_2$ converted by 1 g of platinum group elements in the amorphous alloy catalyst for 1 h q |
|---|---|
| 103 | 0.07 |
| 157 | 0.62 |
| 199 | 2.14 |
| 247 | 3.98 |
| 299 | 4.06 |

Example 2

Raw alloys, whose nominal compositions are shown in Table 2, were prepared by argon arc melting of mixtures of commercial metals. After remelting of the raw alloys under an argon atmosphere, amorphous alloys were prepared by the rotation wheel method by using the apparatus shown in the figure. The amorphous alloys thus prepared were 0.01-0.05 mm thick, 1-3 mm wide and 3-20 m long ribbons. The formation of an amorphous structure was confirmed by X-ray diffraction.

The highly active metallic catalysts were obtained by surface activation treatment of these alloys by immersion in 1.2-46% HF solutions at ambient temperature. The reactor tube was prepared by placing 0.5 g of the metallic catalysts of 5 cm length in a glass tube of 8 mm inner diameter and was placed in an electric furnace.

The reactant gas consisting of 4.96 % of $CO_2$ and 20 % of $H_2$ in $N_2$ was passed through the reactor tube with a flow rate of 15 ml/min. The amounts of $CO_2$, $CH_4$ and $C_2H_4$ in the gas passed through the reactor tube were analyzed by gas chromatography.

Table 2 shows the reaction temperature and conversion.

## Table 2

| Alloy (at%) | Mass of $CO_2$ converted by 1 g of platinum group elements in the amorphous alloy catalysts for 1 h (g) | |
|---|---|---|
| Reaction Temperature | 150°C | 200°C |
| Ni-40Zr-1.5Rh-1.5Ru | 0.62 | 2.14 |
| Ni-30Ta-2Rh | 0.41 | 1.43 |
| Ni-30Ta-2Pt | 0.35 | 1.12 |
| Ni-30Ta-2Ir | 0.33 | 1.08 |
| Ni-30Ta-2Pd | 0.34 | 1.10 |
| Ni-30Ta-2Ru | 0.30 | 0.99 |
| Ni-30Ta-3Rh | 0.42 | 1.45 |
| Ni-40Nb-2Rh | 0.44 | 1.50 |
| Ni-40Nb-2Ru | 0.3 | 1.10 |
| Ni-40Zr-5Rh | 0.42 | 1.42 |
| Ni-40Ta-30Nb-10Rh | 0.50 | 1.87 |
| Ni-10Ta-10Nb-0.5Rh | 0.41 | 1.43 |
| Ni-20Ta-20Nb-10Rh-10Ru | 0.76 | 2.50 |
| Ni-20Co-20Ta-10Nb-2Rh | 0.43 | 1.45 |
| Co-40Nb-3Rh | 0.42 | 1.43 |
| Ni-70Ti-0.5Ru | 0.31 | 1.00 |
| Ni-40Zr-0.25Rh-0.25Pt | 0.55 | 1.98 |
| Ni-20Zr-1Pt | 0.35 | 1.13 |
| Ni-40Ti-40Zr-3Ir | 0.34 | 1.10 |
| Co-20Zr-5Pd | 0.34 | 1.09 |
| Co-20Ti-20Pd | 0.36 | 1.12 |

## Table 2 (continued)

| Alloy (at%) | Mass of $CO_2$ converted by 1 g of platinum group elements in the amorphous alloy catalysts for 1 h (g) | | |
|---|---|---|---|
| | Reaction Temperature | 150°C | 200°C |
| Ni-20Co-40Zr-1Pd-1Rh-1Ru-0.5Pt | | 0.62 | 2.16 |
| Ni-70Ta-10Ti-1Pd | | 0.33 | 1.10 |
| Ni-10Ta-30Nb-20Zr-1Pd | | 0.32 | 1.13 |
| Ni-10Ta-10Nb-20Ti-20Zr-3Ru | | 0.31 | 1.10 |
| Co-30Nb-10Zr-3Ir | | 0.33 | 1.10 |
| Ni-30Co-10Ta-10Nb-10Ti 10Zr-0.25Ir-0.25Rh | | 0.63 | 2.18 |

Consequently, the amorphous alloy catalysts of the present invention have very high activities for conversion to hydrocarbon by the reaction of carbon dioxide with hydrogen.

## Claims

1. Use of a catalyst for conversion of carbon dioxide, said catalyst being produced by
   a) providing an amorphous alloy consisting of
      - 20 to 70 at% of at least one element selected from the group of Nb and Ta, or 20 to 80 at% of at least one element selected from the group of Ti and Zr, or 20 to 80 at% in the total of at least one element selected from the group of Ti and Zr and at most 70 at% of one element selected from the group of Nb and Ta,
      - 0.5 to 20 at% of at least one element selected from the platinum group, and
      - the substantial balance of at least one element selected from the group of Ni and Co; and
   b) immersing said alloy in hydrofluoric acids for activation.

## Patentansprüche

1. Verwendung eines Katalysators zum Umwandeln von Kohlendioxyd, wobei der Katalysator hergestellt ist durch
   a) Schaffen einer amorphen Legierung bestehend aus
      - 20 bis 70 At% von zumindest einem aus der aus Nb und Ta bestehenden Gruppe ausgewählten Element, oder 20 bis 80 At% von zumindest einem aus der aus Ti und Zr bestehenden Gruppe ausgewählten Element, oder insgesamt 20 bis 80 At% von zumindest einem aus der aus Ti und Zr bestehenden Gruppe ausgewählten Element und höchstens 70 At% eines aus der aus Nb und Ta bestehenden Gruppe ausgewählten Elements,
      - 0,5 bis 20 At% von zumindest einem aus der Platin-Gruppe ausgewählten Element, wobei
      - der Rest im wesentlichen aus zumindest einem aus der aus Ni und Co bestehenden Gruppe ausgewählten Element besteht; und
   b) Eintauchen der Legierung in Fluorwasserstoffsäuren zum Aktivieren.

**Revendications**

1.  Utilisation d'un catalyseur pour la conversion du dioxyde de carbone, ledit catalyseur étant produit:

    (a) en prenant un alliage amorphe constitué de:

    - de 20 à 70% en atomes d'au moins un élément choisi dans le groupe formé par Nb et Ta, ou de 20 à 80% en atomes d'au moins un élément choisi dans le groupe formé par Ti et Zr, ou de 20 à 80% en atomes, au total d'au moins un élément choisi dans le groupe formé par Ti et Zr et au plus 70% en atomes d'un élément choisi dans le groupe formé par Nb et Ta,
    - de 0,5 à 20% en atomes d'au moins un élément choisi dans le groupe du platine,
    - le complément étant pratiquement formé d'au moins un élément choisi dans le groupe formé par Ni et Co; et

    b) activation de cet alliage par immersion dans de l'acide fluorhydrique.